# EUROPEAN PATENT APPLICATION

(11) **EP 4 455 297 A1**
(43) Date of publication of application: **30.10.2024**
(21) Application number: 22911077.0
(22) Date of filing: 15.12.2022
(51) Int. Cl.: C12Q 1/04, C12Q 1/24, C12M 1/26, C12M 1/34

(54) **METHOD FOR COLLECTING MICROORGANISMS**

(30) Priority: 22.12.2021 JP 2021208674
(71) Applicant: Horiba Advanced Techno, Co., Ltd., Kyoto-shi, Kyoto 6018551 (JP)
(72) Inventor: NAKAYAMA, Hideki, Kyoto-shi, Kyoto 601-8551 (JP); IRIKURA, Daisuke, Kyoto-shi, Kyoto 601-8551 (JP); OGAWA, Yoko, Kyoto-shi, Kyoto 601-8551 (JP)
(74) Representative: Isarpatent
(86) International application number: PCT/JP2022/046216
(87) International publication number: WO 2023/120369

(57) **Abstract**

An object of the present invention is to more efficiently collect microorganisms present on a surface of an inspection object than before. A microorganism collecting method for collecting microorganisms attached to a surface of an inspection object, the method including: a liquid supply step of supplying a liquid to the surface; and a collecting step of gathering the liquid by a collection member that hardly adsorbs the microorganisms to collect the liquid.

## Description

### Technical Field

The present invention relates to a method for efficiently collecting microorganisms in order to rapidly and quantitatively detect the microorganisms present on a surface of an inspection object with high sensitivity.

### Background Art

For example, in a sterile environment such as a clean room used for handling cultured cells, it is very important that there is no microbial contamination in the environment.

Therefore, before the work in the clean room, for example, it is preferable to perform the work after confirming that contaminant microorganisms as a source of microbial contamination are not present on the surface of a work table or the like.

As a method for confirming the absence of contaminant microorganisms on the surface of the work table, a method is recommended in which the surface of the work table is wiped off with the tip portion of a swab (medical swab) moistened with water, a suspension obtained by swinging the tip portion of the swab in water or a buffer solution in a test tube is applied to a culture medium and cultured to confirm whether or not colonies of microorganisms are generated (Non Patent Literature 1).

However, in this conventional method, for example, culture for 2 to 5 days is required in the case of bacteria, and culture for 5 to 7 days is required in the case of fungi, and thus there is a problem that it takes time to detect contaminant microorganisms.

### Citation List

### Non Patent Literature

Non Patent Literature 1: "Cleanrooms and associated controlled environments-Biocontamination control-Part 1: General principles and methods", Japanese Industrial Standard JIS B9918-1: 2008

### Summary of Invention

### Technical Problem

One of the reasons why it takes time to detect contaminant microorganisms by the conventional method is that the amount of contaminant microorganisms that can be collected from the surface of the work table or the like is small in the first place.

Therefore, the present invention has been made to solve the above-described problem, and an object of the present invention is to provide a method for collecting contaminant microorganisms capable of improving the collecting rate of contaminant microorganisms than before in order to detect contaminant microorganisms more quickly than before.

### Solution to Problem

That is, the method according to the present invention is a method for collecting microorganisms attached to a surface of an inspection object, the method including: a liquid supply step of supplying a liquid to the surface; and a collecting step of collecting the liquid from the surface.

According to such a method for collecting microorganisms, since the liquid is directly supplied to the surface of the inspection object and the liquid is collected, the microorganisms can be efficiently collected as compared with the conventional method in which the tip of the swab is moistened to wipe the microorganisms from the surface.

As a result of careful examination of the conventional method by the present inventors, it has been found that there is a possibility that microorganisms are adsorbed to the tip portion of a swab formed of a nonwoven fabric since microorganisms are collected using the swab in the above-described conventional method. Therefore, it is considered that the collecting rate of microorganisms can be further improved by gathering and collecting the liquid supplied to the surface of the inspection object by a collection member that hardly adsorbs the microorganisms.

As a specific embodiment of the present invention, at least a portion of the collection member in contact with the liquid is made of, for example, a resin such as a natural resin or a synthetic resin. Specific examples of the natural resin include natural rubber and the like. Specific examples of the synthetic resin include at least one selected from the group consisting of polyethylene, polypropylene, silicone resin, and thermoplastic elastomer.

In order to further improve the collecting rate of microorganisms, it is preferable to spray and supply the liquid to the surface.

If the concentration of microorganisms in the liquid can be increased by concentrating the liquid collected from the surface of the inspection object by filtration or the like, the culture time of microorganisms can be further shortened, or microorganisms can be detected without culturing, which is preferable.

As a rapid microorganism test method for detecting microorganisms in a shortest time without culturing the microorganisms, for example, solid phase cytometry, flow cytometry, an immunological method, a nucleic acid amplification method, a bioluminescence method, a microcolony method, an impedance method, a gas measurement method, a fatty acid decomposition method, an infrared absorption spectrum measurement method, a mass spectrometry method, a fingerprint method, high throughput sequencing, and the like are known. Among these rapid test methods, an ATP measurement method for measuring the amount of ATP derived from microorganisms, which is one type of bioluminescence method, is preferable because even a trace amount of microorganisms can be accurately detected.

In the ATP measurement method described above, it is preferable to measure the amount of ATP extracted from the microorganisms after adding an ATP scavenger solution for removing free ATP to the liquid collected from the surface of the inspection object or a concentrate obtained by concentrating the liquid by filtration or the like because only ATP derived from living microorganisms can be measured.

When the microorganisms contained in the liquid or the concentrate are in a spore state with high durability, ATP may not be extracted by the ATP extraction solution described above. Therefore, as long as the amount of ATP extracted from the microorganisms is measured after a germination solution for germinating the contaminant microorganisms in the spore state is added to the liquid or the concentrate, ATP can be extracted from all the microorganisms contained in the liquid containing the microorganisms in the spore state.

The present invention also includes a contaminant microorganism collecting device including a loquid supply unit that supplies a liquid to a surface of an inspection object and a liquid collecting unit that collects the liquid from the surface. Advantageous Effects of Invention

According to the present invention, microorganisms present on a surface of an inspection object can be collected at a higher collecting rate than before. As a result, the culture time for detecting microorganisms can be shortened, or microorganisms can be detected without culturing, and microorganisms can be detected more quickly than before.

### Brief Description of Drawings

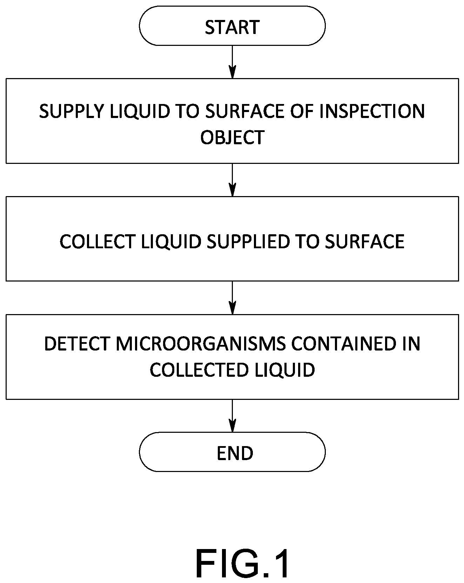

FIG. 1 is a flowchart showing a procedure of a microorganism collecting method according to an embodiment of the present invention.
FIG. 2 is a schematic diagram of a microorganism detection device used in the present embodiment.
FIG. 3 is an overall view of a microorganism detection system using the microorganism collecting method according to the present invention.
FIG. 4 is a schematic diagram showing a concentration device according to another embodiment of the present invention.
FIG. 5 is a schematic diagram showing a filter cassette portion of a concentration device according to another embodiment of the present invention.
FIG. 6 is a schematic view showing a microorganism collecting method according to an example of the present invention.
FIG. 7 is a schematic view showing a conventional microorganism collecting method (comparative example).

### Description of Embodiments

Hereinafter, a method for detecting microorganisms using the microorganism collecting method according to the present invention will be described.

### <Microorganism collecting method>

A microorganism collecting method according to the present embodiment is intended to collect contaminant microorganisms attached to a surface of an inspection object such as a work table in a sterile environment such as a clean room, and includes a liquid supply step of supplying a liquid to the surface and a collecting step of collecting the liquid from the surface.

Examples of the microorganism include protozoa, algae, lichens, fungi, eubacteria, archaea, mold, yeast, bacteria, and the like.

The liquid contains water, and for example, it is preferable that the liquid does not damage microorganisms to be collected such as pure water and a buffer solution. The liquid more preferably contains a surfactant. The liquid may contain an inactivating agent such as lecithin, polysorbate 80, or sodium thiosulfate. The inactivating agent neutralizes and inactivates a decontaminant and a disinfectant present on the surface to be inspected, and suppresses the influence of the decontaminant and the disinfectant on microorganisms contained in the collected liquid.

As the liquid, a sterilized liquid containing no bacterial cells is used. Hereinafter, sterilized tools, containers, and the like that come into contact with the liquid are also used.

In the liquid supply step, the liquid is supplied to the surface by, for example, dropping or spraying. In the present embodiment, droplets are supplied as uniformly as possible to the entire surface by spraying the liquid with a spray.

In the liquid collecting step, the liquid supplied to the surface is collected in a container such as a tube with, for example, a pipette. In the present embodiment, since the liquid is sprayed onto the entire surface as described above, it is preferable to collect the liquid before collecting the liquid droplets supplied to the entire surface.

Therefore, in the present embodiment, the liquid supplied to the surface is gathered by a collection member such as a cell scraper, and then sucked and collected with a pipette.

As the collection member, it is easy and convenient to use a commercially available sterilized cell scraper or the like as described above, but the collection member is not limited thereto, and it is preferable to include a blade (wiper) capable of gathering the liquid on the surface as thoroughly as possible. The method for gathering the liquid is not particularly limited, but for example, the entire inspection region of the surface is rubbed with a blade or the like of the collection member described above without a gap, and microbial cells of microorganisms are gathered with the liquid, for example, at one place or a plurality of places.

When a portion of the collection member in contact with the liquid (for example, a blade) adsorbs microorganisms contained in the liquid by adsorbing the microorganisms in the liquid, the collecting rate of the microorganisms contained in the liquid collected by a pipette decreases.

Therefore, it is preferable that a portion of the collection member in contact with the liquid (for example, a blade) is made of a material that hardly adsorbs the liquid, that is, is that hardly adsorbs microorganisms, or includes a coating layer formed of the material that hardly adsorbs microorganisms on a surface thereof.

As the material that hardly adsorbs microorganisms, for example, resins such as natural resins and synthetic resins can be widely used. Specific examples of the resin include at least one or more substances selected from the group consisting of natural rubber, polyethylene, polypropylene, silicone resin, and thermoplastic elastomer. In particular, even in a case where the surface of the inspection object is not flat but curved like a silicone resin, it is more preferable that the inspection object is deformed along the surface shape.

The above-described coating layer may be further formed on the surface of the blade formed of the material containing a substance that hardly adsorbs microorganisms described above.

Examples of the substance for forming the coating layer include a 2-methacryloyloxyethyl phosphorylcholine polymer and the like.

### <Microorganism detection method>

The method for detecting microorganisms for the liquid collected from the surface in this manner is not particularly limited, but for example, a microorganism detection device for detecting substances derived from microorganisms present in the liquid may be used.

Examples of the microorganism detection device include a device that measures the amount of a substance derived from an organism by analyzing light generated by a substance derived from microorganisms contained in a sample. Hereinafter, an ATP amount measurement device 1 for measuring the amount (amol (= 10⁻¹⁸ mol)) of ATP (adenosine triphosphate) as a substance derived from microorganisms will be described.

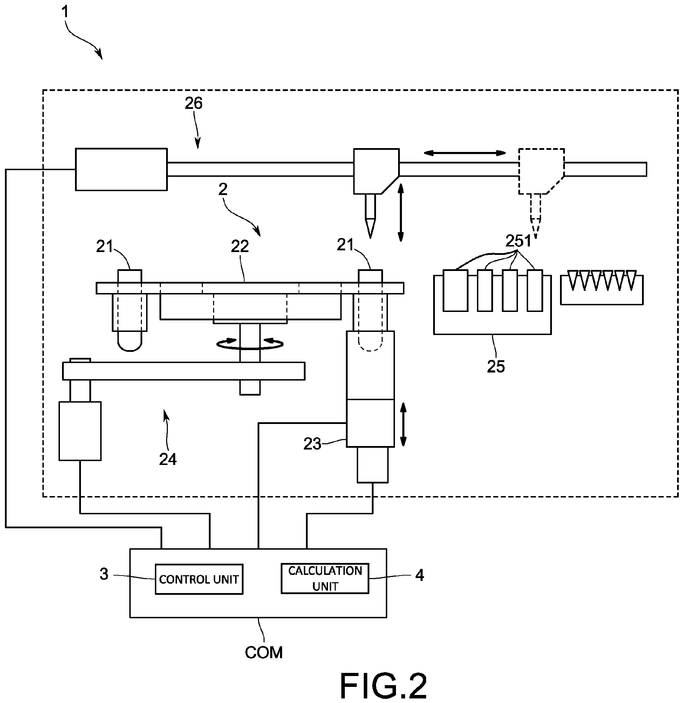

For example, as shown in FIG. 2, the ATP amount measurement device 1 includes a measurement unit 2 that measures the amount of ATP in a sample, a control unit 3 that controls the measurement unit 2, a calculation unit 4 that calculates the amount of ATP based on an output signal from the measurement unit 2, a display unit (not shown) that displays the amount of ATP calculated by the calculation unit 4, and the like.

The measurement unit 2 is a so-called ATP meter, and includes, for example, as shown in FIG. 2, a holder 22 that holds a plurality of containers 21 containing a sample, a photodetector 23 fixed at a predetermined position, a holder drive mechanism 24 that moves the holder 22, a reagent setting unit 25 in which reagent containers 251 containing various reagents including a luminescent reagent that causes ATP to emit light are set, a dispensing mechanism 26 that dispenses various reagents from the reagent containers 251 set in the reagent setting unit 25 into the container 21 held by the holder 22, and a housing that contains them therein (not shown).

As shown in FIG. 2, the photodetector 23 detects light emitted from the sample in the container 21 held by the holder 22, and is, for example, a photomultiplier tube (PMT).

The holder drive mechanism 24 moves the holder 22 to sequentially position the containers 21 held by the holder 22 at detection positions by the photodetector 23.

In the reagent setting unit 25, a reagent container 251 containing a reaction reagent for extracting ATP from the sample and a reagent container 251 containing a luminescent reagent are set. Examples of the reaction reagent include an ATP scavenger solution for scavenging ATP other than living cells (living bacteria) contained in the treated liquid (free ATP), a spore reaction liquid for germinating bacteria in a spore state, an ATP extraction solution for extracting ATP from living cells, and the like.

For example, the dispensing mechanism 26 dispenses each reagent set in the reagent setting unit 25 to the sample contained in each container 21 held by the holder 22.

The liquid collected by the collecting method described above is set as the sample in the holder 22 of the microorganism detection device configured as described above, and measurement is started. The liquid may be fractionated in a predetermined amount from a tube containing the collected liquid, transferred to the container 21 of the microorganism detection device, and then set in the holder 22, or the liquid may be directly collected in a microtube that can be used as the container 21.

When the measurement is started, the control unit 3 controls the dispensing mechanism 26 to dispense each reaction reagent into each container according to a predetermined sequence. As a result, predetermined processing (ATP extraction) is performed on the sample in the container 21.

Specifically, a mixed liquid of an ATP scavenger solution and a spore reaction liquid is dispensed into the sample in the container 21, and the process stands by while maintaining at a predetermined temperature until a reaction of each reagent is completed. Thereafter, an ATP extraction solution is dispensed into the sample in the container, and the process stands by while maintaining at a predetermined temperature until extraction of ATP is completed. The ATP scavenger solution and the spore reaction solution may be separately dispensed instead of the mixed solution of the ATP scavenger solution and the spore reaction solution.

For example, when the ATP scavenger solution and the spore reaction solution are added to the sample, ATP is then extracted with the ATP extraction solution, and a luminescent reagent is added for measurement, it is possible to measure the amount of ATP derived from all contaminant microorganisms in a living state including contaminant microorganisms in a spore state. In addition, if the amount of ATP extracted from the control sample to which the spore reaction liquid is not added at this time is also measured, it is also possible to calculate the amount of ATP derived only from the microorganisms in the spore state.

Thereafter, the control unit 3 controls the holder drive mechanism 24 to move the container 21 to be measured to the detection position. After the container 21 to be measured is moved to the detection position, the control unit 3 controls the dispensing mechanism 26 to introduce the luminescent reagent into the container 21 at the detection position. As a result, light emitted from the sample in the container 21 at the detection position is detected by the photodetector 23, whereby luminescence measurement is performed. Note that, before the luminescence measurement of each container 21, measurement of a blank solution not containing ATP and measurement of a standard solution with a known ATP concentration are performed, and 0 point calibration and span calibration are performed.

A light intensity signal obtained by the photodetector 23 is subjected to arithmetic processing by the calculation unit 4 to calculate the amount of ATP (amol).

For example, the calculation unit 4 subtracts the "light intensity signal obtained before adding the luminescent reagent to the sample" from the "light intensity signal obtained after adding the luminescent reagent" to remove the luminescence in the container 21, and calculates a value related to the amount of a substance derived from an organism.

The user determines whether or not contaminant microorganisms are present in the sample based on the amount of ATP calculated as described above. Specifically, for example, a predetermined threshold value may be set in advance for the ATP concentration in the sample obtained from the amount of ATP calculated as described above, and when the ATP concentration in the sample exceeds this threshold value, it may be determined that contaminant microorganisms are present.

### <Effects of present embodiment>

According to the microorganism collecting method and the microorganism detection method of the present embodiment configured as described above, since the liquid is sprayed onto the entire surface and gathered and collected with a cell scraper or the like, the collecting rate of microorganisms can be greatly improved as compared with the conventional method.

One of the reasons why the collecting rate of microorganisms can be improved is that since the liquid is sprayed and supplied to the entire surface, it is possible to suppress destruction of microorganisms due to friction or the like at the time of wiping.

Further, in the present embodiment, since the liquid is collected using a collection member such as a cell scraper in which a material of a portion in contact with the liquid is formed of the liquid and a material that hardly adsorbs microorganisms, it is considered that the microorganisms adsorbed to the collection member can be reduced as compared with the conventional case of using a swab moistened with water.

Since the method of measuring ATP derived from microorganisms is adopted as the detection method, microorganisms can be detected with high sensitivity even when the number of contaminant microorganisms in a sample is very small.

In addition, it is not necessary to culture microorganisms according to this method, so that microorganism detection can be performed in a short time.

Furthermore, in the present embodiment, the measurement unit 2 of the ATP amount measurement device 1 subtracts the light intensity signal obtained before the reaction of the sample and the luminescent reagent from the light intensity signal obtained after the reaction of the sample and the luminescent reagent to calculate the value related to the amount of ATP, and thus, it is not necessary to detect the "luminescence intensity in a state where the luminescence is suppressed" as in the conventional case. As a result, the measurement time of the sample can be shortened. As a result, it is possible to further shorten the time required for the detection of microorganisms.

### <Other embodiments>

Note that the present invention is not limited to the above embodiment.

The microorganism collecting method according to the present invention may be performed by a person as described above, but may be performed using, for example, a microorganism collecting device as described below.

### <Microorganism collecting device>

Examples of the microorganism collecting device according to the present invention include a device for collecting contaminant microorganisms attached to a surface of an inspection object such as a work table in a sterile environment such as a clean room, and including a liquid supply unit that supplies a liquid to the surface of the inspection object and a liquid collecting unit that collects the liquid from the surface.

The liquid supply unit, for example, collects a predetermined amount of the liquid from a liquid holder that contains the liquid and sprays the liquid onto the surface, and may include a nozzle that ejects the liquid, a pipe that forms a flow path for transporting the liquid from the liquid holder to the nozzle, a pump provided on the flow path, and the like.

The liquid collecting unit sucks the liquid supplied to the surface and collects the liquid in a container such as a tube or a bottle, and may include, for example, a suction port for sucking the liquid, a pipe that forms a flow path for transporting the liquid sucked from the suction port to the container, a pump provided on the flow path, and the like.

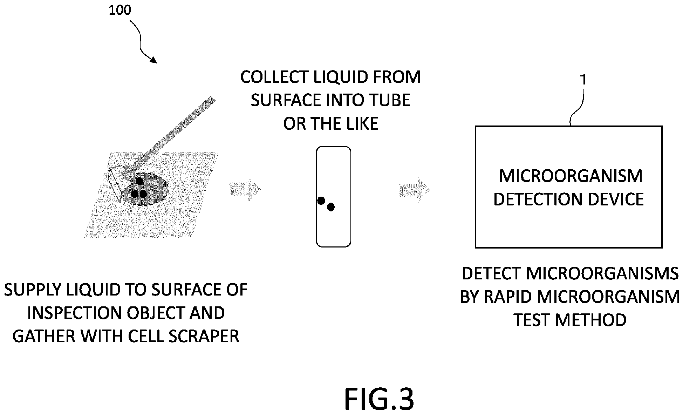

For example, as shown in FIG. 3, it may be configured as a microorganism detection system 100 that detects microorganisms in a person or a liquid collected by the microorganism collecting device by the microorganism detection device using the above-described microorganism collecting method.

A concentration step of filtering or the like the liquid collected from the surface of the inspection object may be further provided, and the microorganisms to be detected may be detected after the microorganisms are concentrated.

This concentration step may be manually performed by the user using a filter as described later, but the microorganism detection system 100 may include a concentration device that concentrates the liquid by filtering.

The concentration device functions as, for example, a concentration unit that obtains a concentrate obtained by concentrating contaminant microorganisms, and concentrates using a filter FC1 that does not allow the contaminant microorganisms to permeate therethrough.

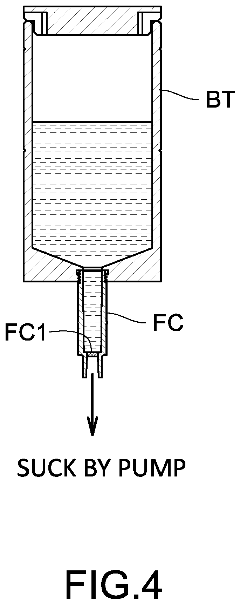

For example, as shown in FIG. 4, the concentration device includes a bottle BT that stores a sample, a cartridge that is detachably attached to a lower end opening of the bottle BT and includes a filter FC1 in which a fine hole with a size that does not transmit contaminant microorganisms is formed (specimen tube FC), and a pump that sucks an internal liquid from a lower end portion of the specimen tube (not shown).

In a state where the upper end portion of the cartridge is inserted into the lower end opening of the bottle BT, the liquid inside the specimen tube FC is sucked from the lower end portion of the specimen tube FC by a pump or the like, so that the sample can be concentrated on the filter FC1 of the specimen tube FC.

In order to remove substances other than contaminant microorganism cells as much as possible, the concentrate may be washed by adding distilled water, an appropriate buffer solution, or the like onto the filter FC1. This washing step may be performed by a concentration device or may be manually performed by the user.

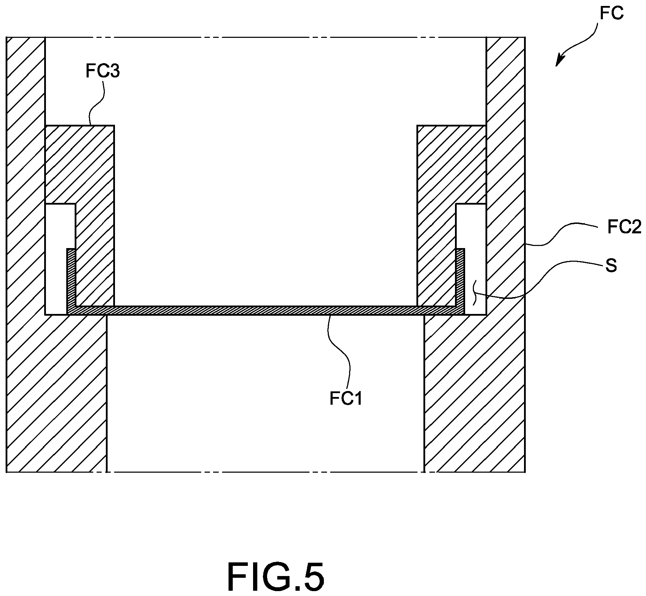

For example, as shown in FIG. 5, the specimen tube FC includes a cylindrical tube body FC2 with both ends opened, and a filter cassette FC3 arranged so as to be in contact with an inner peripheral surface of the tube body FC2 and arranging the filter FC1 in the middle of the tube body FC2 in a direction perpendicular to the axial direction of the tube body FC2. When the filter cassette FC3 is attached to the inner peripheral surface of the tube body FC2, a slight gap S may be formed between the filter cassette FC3 and the inner peripheral surface of the tube body FC2. When a sample is supplied into the specimen tube FC in a state where the gap S is formed, there is a possibility that a part of the sample that has passed through the filter enters the gap S. As a result, when the sample contains a component that affects luminescence measurement, accurate measurement is inhibited.

Therefore, before the sample is supplied to the specimen tube FC, it is preferable that a gap filling liquid such as a buffer solution or distilled water is circulated to allow the gap filling liquid to enter the gap S formed between the filter cassette FC3 and the tube body FC2 in advance. The type of gap filling liquid to enter the gap S in advance may be any type that does not affect ATP measurement, and may be other than the buffer solution or distilled water described above. The liquid preferably has viscosity, and more preferably has viscosity derived from a chemical substance that is not derived from an organism. Specific examples of such a gap filling liquid include an aqueous solution containing a viscous substance such as a polyol. Specific examples of the viscous substance include one or more selected from the group consisting of glycerol, polyethylene glycol, polyoxyethylene, polyoxyethylene sorbitan monooleate, polyacrylamide, and the like. In order to make it easier for these gap filling liquids to enter the above-described gap S, the gap filling liquid may contain a chemical agent such as a surfactant.

In the above-described embodiment, the case of collecting contaminant microorganisms in a clean room has been described, but the microorganism collecting method according to the present invention is not limited thereto, and can be applied to, for example, various microorganisms attached to a dried surface. In particular, when it is desired to detect the presence of microorganisms but it is desired to increase the collecting rate as much as possible because only a trace amount of microorganisms are present, the effect can be remarkably exhibited.

The method for detecting microorganisms is not limited to the ATP measurement as described above, and may be, for example, a method using proliferation as an index, such as culture on a plate and confirmation of the number of colonies as in the conventional case. In addition, it is also possible to use a method of directly measuring microorganisms such as a cytometry method.

The determination as to whether or not the microorganisms are present in the sample may be performed by the user as described above, or may further include a determination unit that determines whether or not the microorganisms are present from the amount of ATP calculated by the calculation unit.

In addition, the present invention is not limited to the above embodiment, and it goes without saying that various modifications can be made without departing from the gist of the present invention.

### Examples

Hereinafter, the present invention will be described more specifically, but the present invention is not limited thereto.

In this example, it was confirmed that the microorganism collecting rate for the microorganism collecting method according to the present invention was improved as compared with that for the conventional method. The experiment was performed according to the following procedure.

### <Example>

First, the surface of a stainless steel plate of 10 cm square was washed and dried.

A predetermined number of Bacillus subtilis was seeded on the surface of the stainless steel plate. Specifically, a predetermined amount of a bacterial suspension with a known number of bacteria contained in 1 ml was dropped and dried.

Thereafter, 0.3 mM buffer solution (pH 7.2, sterilized) containing 0.05% Tween 80 was sprayed over the entire surface, the entire surface of the stainless steel plate was wiped with a sterilized cell scraper to gather the buffer solution, and the collected buffer solution was collected into a sterilized microtube with a pipette.

The number of bacterial cells of Bacillus subtilis contained in the buffer solution collected in the microtube was measured by ATP measurement or colony formation on an agar medium.

In addition, an agar medium was also supplied onto the stainless steel plate after wiping off the buffer solution, and the number of bacterial cells remaining on the stainless steel plate was confirmed by colony formation.

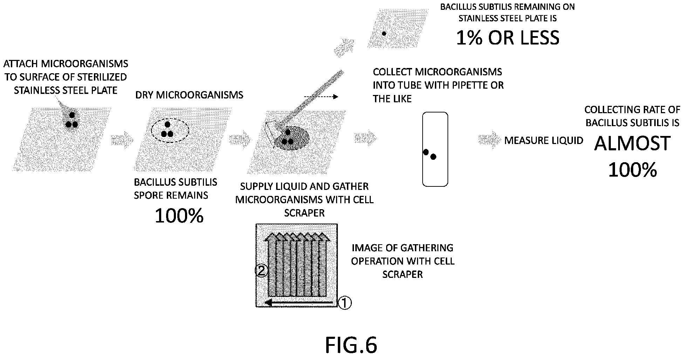

The procedure of Example is summarized in FIG. 6.

### <Comparative Example>

In the same procedure as in Example, the surface of the stainless steel plate on which approximately the same number of Bacillus subtilis as that in Example was seeded was collected by the conventional method.

Specifically, the entire surface of the stainless steel plate was wiped with a swab whose tip was moistened with sterile water, and then the tube was shaken by vortexing in a state where the swab was disposed so that the tip was immersed in sterile water contained in the tube to obtain a suspension.

The number of bacterial cells contained in each of the suspension thus obtained and the stainless steel plate after wiping off Bacillus subtilis was measured in the same procedure as in Example.

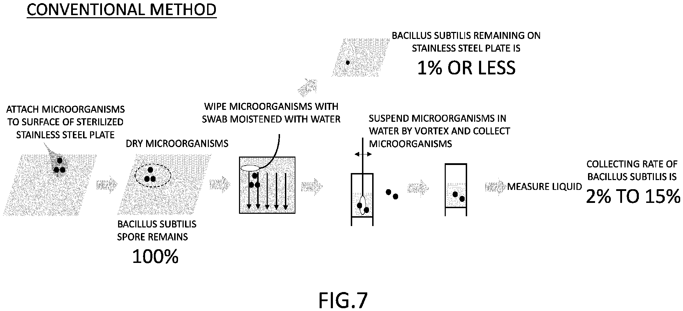

The procedure of Comparative Example is summarized in FIG. 7.

### <Results and discussion>

As a result of this experiment, in Example, almost 100% of Bacillus subtilis seeded on the surface of the stainless steel plate could be collected in all the experiments performed a plurality of times. In Example, even when sterile water was used instead of the buffer solution, 50% or more of bacterial cells were successfully collected.

On the other hand, in Comparative Example, only 2% to 15% of the Bacillus subtilis seeded on the surface of the stainless steel plate was collected.

From this result, it can be seen that the microorganism collecting method according to the present invention could greatly improve the collecting rate of microorganisms as compared with the conventional method.

The ratio of Bacillus subtilis remaining on the surface of the stainless steel plate after collecting Bacillus subtilis was as very small as about 1% by any method.

From these results, in the conventional method, Bacillus subtilis can be wiped off from the surface of the stainless steel plate, but there is a possibility that Bacillus subtilis cannot be extracted from the swab in the step of suspending the bacterial cells collected at the tip of the swab in sterile water in the tube.

In this regard, according to the present invention, since a swab that easily adsorbs sterile water is not used, it can be presumed that the number of microorganisms adsorbed to the tool during the collecting is reduced, and a higher collecting rate can be realized.

In addition, as another cause of the low collecting rate in the conventional method, when the surface of the stainless steel plate is wiped off with a swab whose tip is moistened, there is a case where sufficient liquid is not present at the tip of the swab, so that there is a possibility that friction is generated by direct contact between the swab and the stainless steel surface, and Bacillus subtilis is destroyed by this frictional force.

Therefore, in the above-described Example, the same amount of sterile water was collectively dropped at one place on the surface of the stainless steel plate instead of spraying the sterile water to the entire surface of the stainless steel plate, and the droplet was collected after wiping the surface of the stainless steel plate with a cell scraper such that the droplet moved on the entire surface of the stainless steel plate. As a result, it was found that the collecting rate was about 35% while the collecting rate was sufficiently higher than that in the conventional method.

When the liquid is dropped to one place and the front surface to be inspected is wiped off, the surface of the stainless steel may be scraped in a state in which the liquid is not present between the scraper and the surface of the stainless steel while the liquid is sufficiently present as compared with the conventional case, and there is a possibility that Bacillus subtilis has been broken by friction in such a case. Therefore, from the viewpoint of reducing friction, it has been found that it is more preferable that the liquid supplied to the surface to be inspected is uniformly supplied to the entire surface by a method such as spraying.

## Claims

1. A microorganism collecting method for collecting microorganisms attached to a surface of an inspection object, the method comprising:
a liquid supply step of supplying a liquid to the surface; and
a collecting step of gathering the liquid by a collection member that hardly adsorbs the microorganisms to collect the liquid from the surface.

2. The microorganism collecting method according to claim 1, wherein at least a portion of the collection member in contact with the liquid is formed of at least one or more substances selected from a group consisting of polyethylene, polypropylene, a silicone resin, and a thermoplastic elastomer.

3. The microorganism collecting method according to claim 1 or 2, wherein in the liquid supply step, the liquid is sprayed onto the surface.

4. The microorganism collecting method according to any one of claims 1 to 3, further comprising a concentration step of concentrating the collected liquid.

5. A microorganism detection method for detecting microorganisms present in the liquid collected by the microorganism collecting method according to any one of claims 1 to 4 by a rapid microorganism test method.

6. The microorganism detection method according to claim 5, wherein the rapid microorganism test method measures an amount of ATP present in the liquid.

7. The microorganism detection method according to claim 6, wherein an ATP scavenger solution that removes free ATP is added to the liquid or a concentrate obtained by concentrating the liquid, then an ATP extraction solution is added to the liquid or the concentrate, and the amount of extracted ATP is measured.

8. The microorganism detection method according to claim 6, wherein a germination solution for germinating the microorganisms in a spore state is added to the liquid or a concentrate obtained by concentrating the liquid, then an ATP extraction solution is added to the liquid or the concentrate, and the amount of extracted ATP is measured.

9. A microorganism detection system for collecting microorganisms attached to a surface of an inspection object and detecting the microorganisms, the system comprising:
a microorganism detection device that detects microorganisms contained in a liquid collected from the surface by gathering the liquid by a collection member that hardly adsorbs the microorganisms after the liquid is supplied to the surface.
